# EUROPEAN PATENT APPLICATION

(11) **EP 4 699 572 A1**
(43) Date of publication of application: **25.02.2026**
(21) Application number: 24792077.0
(22) Date of filing: 18.04.2024
(51) Int. Cl.: A61F 2/24

(54) **OUTER CATHETER, CONVEYING ASSEMBLY AND CONVEYING SYSTEM**

(30) Priority: 21.04.2023 CN 202310436708
(71) Applicant: Shanghai MicroPort CardioFlow Medtech Co., Ltd., Shanghai 201203 (CN)
(72) Inventor: YU, Wenxia, Shanghai 201203 (CN); ZHANG, Pinghai, Shanghai 201203 (CN); HUANG, Qingqing, Shanghai 201203 (CN); CHEN, Guoming, Shanghai 201203 (CN)
(74) Representative: Patentship Patentanwaltsgesellschaft mbH
(86) International application number: PCT/CN2024/088564
(87) International publication number: WO 2024/217492

(57) **Abstract**

The present application relates to an outer catheter, a delivery component and a delivery system. A main catheter body comprises an axial lumen and includes an outer-catheter bendable section and an outer-catheter push section along an axial direction. The outer-catheter bendable section has a first axially bend-resistant portion extending along the axial direction. The first axially bend-resistant portion is configured to resist bending of the outer-catheter bendable section along an axial plane where the first axially bend-resistant portion is located. The outer-catheter push section has a second axially bend-resistant portion extending along the axial direction. The second axially bend-resistant portion is configured to resist bending of the outer-catheter push section along an axial plane where the second axially bend-resistant portion is located. An angle may be formed between the axial planes of the first and second axially bend-resistant portions. With this arrangement, a circumferential angular deflection can be created between the first and second axially bend-resistant portions of the outer catheter to allow the two to bend in different planes, enabling three-dimensional bending of the outer catheter, and smooth passage through different vessel lumens of different shapes in the body.

## Description

### TECHNICAL FIELD

The present application relates to the field of medical devices and, in particular, to an outer catheter, a delivery component and a delivery system.

### BACKGROUND

Heart valve replacement, such as aortic valve replacement, mitral valve replacement, tricuspid valve replacement and pulmonary valve replacement, involves delivering, positioning and releasing a prosthetic valve at a target site through a catheter of a delivery system to replace the native valve. Nowadays, this technique has attracted extensive attention in the field of valvular heart disease treatment. For example, transcatheter aortic valve replacement (TAVR) can be used to treat aortic valve disease without surgically opening the chest or stopping the heartbeat, which may cause significant trauma to a patient.

Catheters in existing delivery systems for use in TAVR procedures are not actively steerable three-dimensionally. When navigating through the aortic arch, the catheter of the delivery system relies on passive bending by the reaction force from the vascular wall, thereby advancing through the aortic arch. However, this may cause some damage to the blood vessel wall, possibly leading to certain vascular complications. Further, during the subsequent valve crossing and coaxial adjustment of TAVR, the lack of active steerability of the catheter of the delivery system makes it difficult to ensure that the annulus is in coplanar with the aortic arch This complicates coaxial adjustment, affecting release and anchoring of the valve.

Therefore, how to achieve an accurate coaxial release of the valve stent within the three-dimensional arch through steerable 3D spatial bending control remains a critical technical challenge in this field.

### SUMMARY OF THE INVENTION

In view of the problem described above, it would be desirable to provide an outer catheter, a delivery component and a delivery system.

An outer catheter for use in a delivery system comprises:
a main catheter body comprising an axial lumen, wherein the main catheter body comprises an outer-catheter bendable section and an outer-catheter push section along an axial direction, wherein the outer-catheter bendable section comprises a first axially bend-resistant portion extending along the axial direction, wherein the first axially bend-resistant portion is configured to resist a bending of the outer-catheter bendable section along an axial plane where the first axially bend-resistant portion is located, wherein the outer-catheter push section comprises a second axially bend-resistant portion extending along the axial direction, wherein the second axially bend-resistant portion is configured to resist a bending of the outer-catheter push section along an axial plane where the second axially bend-resistant portion is located, and wherein an angle is formed between the axial plane where the first axially bend-resistant portion is located and the axial plane where the second axially bend-resistant portion is located.

In one embodiment, the outer-catheter bendable section and the outer-catheter push section are an integrally formed structure, wherein a proximal end of the outer-catheter bendable section is fixedly joined to a distal end of the outer-catheter push section.

Alternatively, the outer-catheter bendable section and the outer-catheter push section are separate structures, wherein the proximal end of the outer-catheter bendable section is rotatably coupled to the distal end of the outer-catheter push section, and wherein based on a fixed-axis rotation between the outer-catheter bendable section and the outer-catheter push section, a planar angle between the axial planes where the first and second axially bend-resistant portions are located can be adjusted.

In one embodiment, the proximal end of the outer-catheter bendable section and the distal end of the outer-catheter push section are provided with a pivot-adjusting slot and a mating pivot-locking element, respectively, wherein the pivot-adjusting slot extends circumferentially, wherein the pivot-locking element slides along the pivot-adjusting slot to adjust an angle of the fixed-axis rotation between the outer-catheter bendable section and the outer-catheter push section.

In one embodiment, the outer-catheter bendable section has two first axially bend-resistant portions that are symmetric about a central axis of the outer-catheter bendable section.

Alternatively or additionally, the outer-catheter push section has two second axially bend-resistant portions that are symmetric about a central axis of the outer-catheter push section.

In one embodiment, a surface of the outer-catheter bendable section may comprise an outer-catheter bend-facilitating portion extending along the axial direction, wherein the outer-catheter bend-facilitating portion has no overlap with the axial plane where the first axially bend-resistant portion is located, and wherein the outer-catheter bend-facilitating portion is configured to at least allow the outer-catheter bendable section to bend along a direction perpendicular to the axial plane where the first axially bend-resistant portion is located.

In one embodiment, the outer-catheter bendable section may be provided with two outer-catheter bend-facilitating portions that are in symmetric about a central axis of the outer-catheter bendable section.

In one embodiment, each of the outer-catheter bend-facilitating portions may be provided with a plurality of first hollows, wherein the first hollow comprises a first arc-shaped slot and two first circular slots located at opposite ends of the first arc-shaped slot, wherein the first arc-shaped slot extends circumferentially and is perpendicular to the central axis of the outer-catheter bendable section, wherein the plurality of the first hollows are arranged along the axial direction of the outer-catheter bendable section, and wherein a region between the two outer-catheter bend-facilitating portions constitutes the first axially bend-resistant portion.

In one embodiment, the outer-catheter bend-facilitating portions may be provided with a plurality of second arc-shaped slots that extends circumferentially and is perpendicular to the central axis of the outer-catheter bendable section, and wherein the plurality of second arc-shaped slots are arranged along the axial direction of the outer-catheter bendable section.

Alternatively or additionally, the first axially bend-resistant portion may be provided with at least two lines of axial straight holes extending along the axial direction, wherein a bend-resistant backbone for resisting the bending of the outer-catheter bendable section is provided between the two lines of axial straight holes.

In one embodiment, each line of axial straight hole comprises a plurality of hole units that are axially spaced apart.

In one embodiment, each of the outer-catheter bend-facilitating portions may be provided with a plurality of arc-shaped cutting grooves that extends circumferentially and is perpendicular to the central axis of the outer-catheter bendable section, wherein the plurality of arc-shaped cutting grooves are arranged along the axial direction of the outer-catheter bendable section, and wherein the plurality of arc-shaped cutting grooves in the two outer-catheter bend-facilitating portions are partially alternated along the axial direction.

In one embodiment, a proximal end of the outer-catheter bendable section comprises a diameter-varying segment that is coupled to a distal end of the outer-catheter push section, wherein a diameter of the diameter-varying segment of the outer-catheter push section gradually decreases from a distal end to a proximal end.

Alternatively or additionally, the outer-catheter bendable section may comprise an outer-catheter outer layer and an outer-catheter inner layer.

A delivery component comprises:
the outer catheter as defined above;
an inner catheter comprising a guide lumen, wherein the inner catheter is movably mounted in an axial lumen of the outer catheter;
a pull wire coupled to at least one of the outer catheter and the inner catheter; and
a stent body mounted with a valve, wherein the stent body and the valve are configured to be mounted between the inner catheter and the outer catheter.

In one embodiment, the delivery component may comprise:
a stent retainer provided at a distal end of the inner catheter; and/or
an inner core tube comprising a core-tube lumen, wherein a proximal end of the inner core tube communicates with the distal end of the inner catheter, wherein at least one of the guide lumen and the core-tube lumen is configured for passage of a guide wire therethrough.

In one embodiment, the stent retainer may comprise an inner bore in communication with the guide lumen.

Alternatively or additionally, the stent retainer may comprise a wire fastener.

Alternatively or additionally, the stent retainer may comprise a stent retention member.

Alternatively or additionally, a distal guide member may be provided at a distal end of the inner core tube.

In one embodiment, at least one axial section of the inner catheter may comprise an inner-catheter axially bend-resistant portion extending along an axial direction, and wherein the inner-catheter axially bend-resistant portion is configured to resist a bending of the inner catheter along an axial plane where the inner-catheter axially bend-resistant portion is located.

In one embodiment, the inner catheter may comprise two inner-catheter axially bend-resistant portions that are symmetric about a central axis of the inner catheter.

In one embodiment, the inner catheter may comprise, an inner-catheter bendable section and an inner-catheter push section along the axial direction, wherein a proximal end of the inner-catheter bendable section is coupled to a distal end of the inner-catheter push section, and the inner-catheter axially bend-resistant portion is provided at least in the inner-catheter bendable section.

In one embodiment, a distal end of the pull wire may be coupled to a distal end of the inner-catheter bendable section.

Alternatively, the pull wire may be threaded through the guide lumen of the inner catheter.

Alternatively, at least one axial section of the inner catheter may comprise a pulling channel, wherein the pull wire is threaded through the pulling channel of the inner catheter.

In one embodiment, the inner-catheter bendable section may comprise an inner-catheter outer layer, an inner-catheter inner layer and a reinforcing layer between the inner-catheter outer layer and the inner-catheter inner layer.

In one embodiment, a pulling channel may be provided between the inner-catheter outer layer and the reinforcing layer, or a pulling channel is provided in an inner wall of the inner-catheter inner layer.

In one embodiment, a surface of the inner-catheter bendable section may comprise an inner-catheter bend-facilitating portion extending along the axial direction, wherein the inner-catheter bend-facilitating portion has no overlap with the axial plane where the inner-catheter axially bend-resistant portion is located, and wherein the inner-catheter bend-facilitating portion is configured to at least allow the inner-catheter bendable section to bend along a direction perpendicular to the axial plane where the inner-catheter axially bend-resistant portion is located.

In one embodiment, the inner-catheter bendable section may be provided with two inner-catheter bend-facilitating portions that are symmetric about a central axis of the inner-catheter bendable section.

In one embodiment, the inner-catheter bend-facilitating portion may comprise a plurality of second hollows comprising a third arc-shaped slot and two second circular slots located at opposite ends of the third arc-shaped slot, wherein the third arc-shaped slot extends circumferentially and is perpendicular to the central axis of the inner-catheter bendable section, and wherein the plurality of the second hollows are arranged along the axial direction of the inner-catheter bendable section, and wherein a region between the two inner-catheter bend-facilitating portions constitutes the inner-catheter axially bend-resistant portion.

In one embodiment, the inner-catheter bendable section may comprise a plurality of joint units rotatably articulated along the axial direction, wherein a fixed-axis pivot part is provided between adjacent joint units, wherein the fixed-axis pivot part is located in the inner-catheter axially bend-resistant portion, wherein a rotation gap is provided between adjacent joint units, and wherein the rotation gap is provided in the inner-catheter bend-facilitating portions.

In one embodiment, the fixed-axis pivot part comprises an axle and a hole which are provided in adjacent joint units.

Alternatively, the fixed-axis pivot part comprises a first rotatable engagement structure and a second rotatable engagement structure which are provided on adjacent joint units, wherein the first rotatable engagement structure comprises a first arc-shaped slide slot, a first arc-shaped fastening arm and a central fastening slot, wherein the second rotatable engagement structure comprises a second arc-shaped slide slot, a second arc-shaped fastening arm and a central fastening head, wherein the first arc-shaped fastening arm is slidably mounted along the second arc-shaped slide slot, wherein the second arc-shaped fastening arm is slidably mounted along the first arc-shaped slide slot, and wherein the central fastening head is rotatably mounted in the central fastening slot.

A delivery system comprises:
the outer catheter as defined above; or the delivery component as defined above.

In the outer catheter, delivery component and delivery system as defined above, a circumferential angular deflection may be created between the first and second axially bend-resistant portions of the outer catheter to allow the outer-catheter bendable section and the outer-catheter push section of the outer catheter to bend in different planes, enabling flexible three-dimensional bending of the outer catheter and smooth passage through lumens of different shapes in the body, such as the aortic arch. Based on active three-dimensional steerability of the outer catheter, a valvular annulus in the same plane as the aortic arch can be maintained, facilitating coaxial adjustment and release of a valve stent, thereby significantly improving surgical outcomes.

### BRIEF DESCRIPTION OF THE DRAWINGS

Fig. 1 schematically illustrates an outer catheter in a first bent configuration, according to an embodiment of the present application.
Fig. 2 schematically illustrates an outer catheter in a second bent configuration, according to an embodiment of the present application.
Fig. 3 schematically illustrates a bending rang of an outer catheter according to an embodiment of the present application.
Fig. 4 schematically illustrates an outer-catheter bendable section and an outer-catheter push section, which are circumferentially rotatable relative to each other at an angle, according to an embodiment of the present application.
Fig. 5 is a schematic plan view of the outer-catheter bendable section and the outer-catheter push section of Fig. 4 that are circumferentially pivotable relative to each other at an angle.
Fig. 6 is a schematic assembly view of an outer catheter and an inner catheter, according to an embodiment of the present application.
Fig. 7 schematically illustrates an inner catheter, according to an embodiment of the present application.
Fig. 8 schematically illustrates an outer-catheter bendable section, according to an embodiment of the present application.
Fig. 9 schematically illustrates an outer-catheter bendable section, according to another embodiment of the present application.
Fig. 10 schematically illustrates an outer-catheter bendable section, according to yet another embodiment of the present application.
Fig. 11 schematically illustrates a main catheter body, an outer-catheter outer layer and an outer-catheter inner layer, according to an embodiment of the present application.
Fig. 12 schematically illustrates an inner-catheter outer layer and an inner-catheter inner layer, according to an embodiment of the present application.
Fig. 13 shows a schematic perspective assembly view of an inner-catheter bendable section and a stent retainer, according to an embodiment of the present application.
Fig. 14 shows a schematic plan assembly view of an inner-catheter bendable section and a stent retainer, according to an embodiment of the present application.
Fig. 15 schematically illustrates a stent retainer, according to an embodiment of the present application.
Fig. 16 schematically illustrates an inner-catheter bendable section, according to an embodiment of the present application.
Fig. 17 schematically illustrates an inner-catheter bendable section, according to another embodiment of the present application.
Fig. 18 schematically illustrates an inner-catheter bendable section, according to yet another embodiment of the present application.
Fig. 19 schematically illustrates a pull wire threaded through a guide lumen, according to yet another embodiment of the present application.
Fig. 20 schematically illustrates a pull wire threaded through a pulling channel, according to an embodiment of the present application.
Fig. 21 schematically illustrates a pull wire threaded through a pulling channel, according to another embodiment of the present application.

### List of Reference Numerals

1000, outer catheter; 2000, inner catheter; 3000, pull wire; 4000, stent retainer; 5000, inner core tube;
1100 main catheter body; 1200, outer-catheter bendable section; 1300, outer-catheter push section;
1100a, outer-catheter outer layer; 1100b, outer-catheter inner layer; 1200a, pivot-adjusting slot; 1200b, pivot-locking element; 1200c, sealing element; 1210, first axially bend-resistant portion; 122, outer-catheter bend-facilitating portion; 1310, second axially bend-resistant portion;
1221, first hollow; 1222, second arc-shaped slot; 1223 arc-shaped cutting groove;
1221a, first arc-shaped slot; 1221b, first circular slot; 1222a, axial straight hole;
4100, inner bore; 4200, wire fastener; 4300, stent retention member; 4400, distal guide member;
2100, inner-catheter bendable section; 2200, inner-catheter push section;
2100a, inner-catheter outer layer; 2100b, inner-catheter inner layer; 2100c, reinforcing layer; 2100d, guide lumen;
2110, inner-catheter axially bend-resistant portion; 2120, inner-catheter bend-facilitating portion; 2130, pulling channel;
2121, second hollow; 2122, joint unit;
2121a, third arc-shaped slot; 2121b, second circular slot; 2122a, fixed-axis pivot part; 2122b, rotation gap;
2122a1, first arc-shaped slide slot; 2122a2, first arc-shaped fastening arm; 2122a3, central fastening slot; 2122a4, second arc-shaped slide slot; 2122a5, second arc-shaped fastening arm; 2122a6, central fastening head.

### DETAILED DESCRIPTION

The above objects, features and advantages of the present application will become more apparent and readily appreciated upon reading the following detailed description of a few specific embodiments thereof with reference to the accompanying drawings. In the following description, numerous details are set forth in order to provide a thorough understanding of the application. However, the present application may be practiced in many other forms than those described herein, and those skilled in the art can make similar improvements without deviating from the spirit of the invention. Accordingly, the present application is not limited to the specific embodiments disclosed below.

It will be understood that terms, such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. may be used herein to describe directional or positional relationships based on the orientations shown in the figures. They are intended merely to facilitate and simplify the explanation of the application and do not indicate or imply that the stated components or elements have to comprise, or be constructed or operated in, particular orientations. Therefore, they are not to be construed as limiting the present application.

In addition, when used herein, the terms "first", "second" and the like are intended only for illustration and are not to be construed as denoting or implying relative importance, or as implicitly indicating the number of the referenced items. Accordingly, defining an item with "first" or "second" is an explicit or implicit indication of the presence of one or at least two such items, unless the context clearly dictates otherwise. When used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with an intervening media, or an internal communication or interaction between two elements. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

It should be noted that when a component is referred to as being "secured" to or "disposed" on another component, it may be directly on the other component, or intervening components may be present. When a component is referred to as being "connected" or "coupled" to another component, it can be directly connected or coupled to the other component, or intervening components may be present. As used herein, the terms "vertical", "horizontal", "upper", "lower", "left", "right" and the like are merely illustrative and do not represent the only implementation possible.

In order to more clearly describe the structure of a delivery component, as used herein, the term "distal end" refers to an end farther away from an operator, and the term "proximal end" refers to an end farther closer to the operator, during surgical operation. Unless defined otherwise, all technical and scientific terms used herein have the same meaning as commonly understood by one of ordinary skill in the art to which this application belongs. The terminology used herein is for the purpose of describing particular embodiments only and is not intended to be limiting of the application.

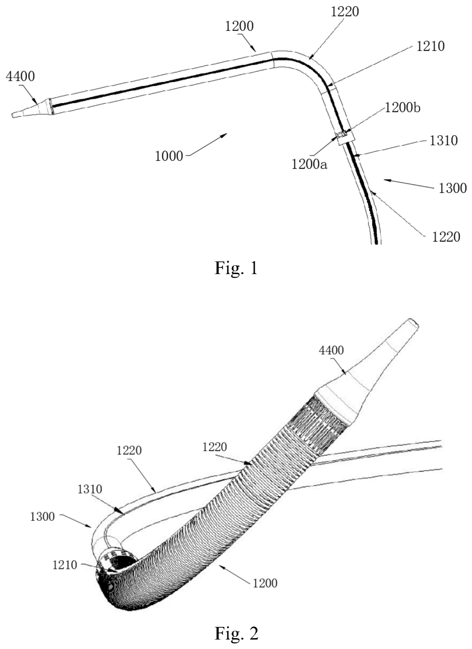

Referring to Fig. 1, in an embodiment of the present application, there is provided an outer catheter 1000 for use in a delivery system. The outer catheter 1000 includes a main catheter body 1100 comprising an axial lumen. The main catheter body 1100 includes, along an axis thereof, an outer-catheter bendable section 1200 and an outer-catheter push section 1300. The outer-catheter bendable section 1200 has a first axially bend-resistant portion 1210 extending along an axis thereof. The first axially bend-resistant portion 1210 is configured to resist bending of the outer-catheter bendable section 1200 along an axial plane where the first axially bend-resistant portion 1210 is located. The outer-catheter push section 1300 has a second axially bend-resistant portion 1310 extending along an axis thereof. The second axially bend-resistant portion 1310 is configured to resist bending of the outer-catheter push section 1300 along an axial plane where the second axially bend-resistant portion 1310 is located. The axial planes of the first and second axially bend-resistant portions 1210, 1310 may form an angle therebetween.

The first and second axially bend-resistant portions 1210, 1310 may be essentially in the form of axially extending linear portions, and may each have a width properly determined as actually needed, without limitations. Both the outer-catheter bendable section 1200 and the outer-catheter push section 1300 may be straight when out of use, and non-straight when in use, as shown in Fig. 1. Accordingly, in the axially straight and non-straight configurations, both the outer-catheter bendable section 1200 and the outer-catheter push section 1300, and hence the axially extending first and second axially bend-resistant portions 1210, 1310, may be linear trajectory or curved trajectory.

It should be noted that each of the aforementioned axial planes refers to a plane passing through a corresponding central axis. Specially, the axial plane in which the first axially bend-resistant portion 1210 is located is a plan passes through both a central axis and a surface of the outer-catheter bendable section 1200, and by "resist bending of the outer-catheter bendable section 1200 in an axial plane of the first axially bend-resistant portion 1210", it is intended to mean that bending of the outer-catheter bendable section 1200 in the axial plane is made difficult, or even impossible at all. Likewise, the axial plane in which the second axially bend-resistant portion 1310 is located is a plan passes through both a central axis and a surface of the outer-catheter push section 1300, and by "resist bending of the outer-catheter push section 1300 in an axial plane of the second axially bend-resistant portion 1310", it is intended to mean that bending of the outer-catheter push section 1300 in the axial plane is made difficult, or even impossible at all.

Limiting bending of the outer-catheter bendable section 1200 and the outer-catheter push section 1300 in said axial planes by the first and second axially bend-resistant portions 1210, 1310, respectively, enables them to more easily bend in other directions, in which their bending is not limited by the first and second axially bend-resistant portions 1210, 1310. Thus, a degree of selectivity for bending directions is imparted to the outer-catheter bendable section 1200 and an inner-catheter push section 2200.

Referring to Fig. 1, the first axially bend-resistant portion 1210 extending along the axis of the outer-catheter bendable section 1200 is coupled to the second axially bend-resistant portion 1310 extending along the axis of the outer-catheter push section 1300. When there is no circumferential deflection between the first and second axially bend-resistant portions 1210, 1310, the first and second axially bend-resistant portions 1210, 1310 restrict bending of the outer-catheter bendable section 1200 and the outer-catheter push section 1300 in the same direction. That is, the outer-catheter bendable section 1200 and the outer-catheter push section 1300 are allowed to bend substantially in a single direction, and their bending is resisted substantially in a different single direction. When subjected to resistance while bending in essentially the same direction, the outer-catheter bendable section 1200 and the outer-catheter push section 1300 are two-dimensionally bendable within a plane.

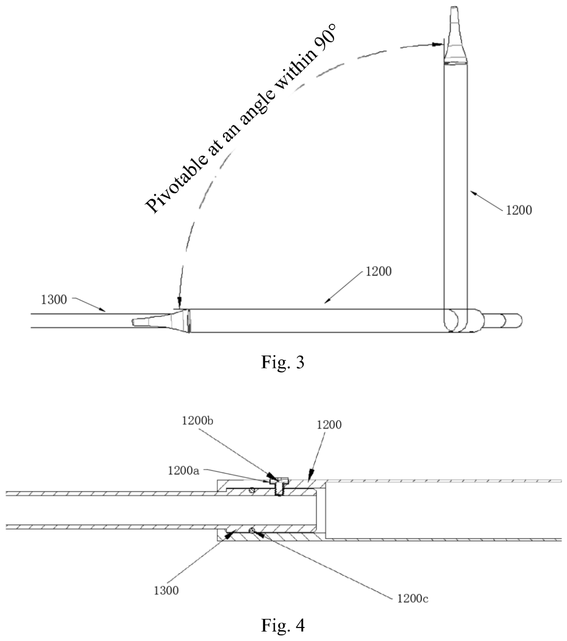

Referring to Fig. 2, when there is a circumferential angular deflection between the first and second axially bend-resistant portions 1210, 1310, the first and second axially bend-resistant portions 1210, 1310 restrict bending of the outer-catheter bendable section 1200 and the outer-catheter push section 1300 in different directions. Thus, the outer-catheter bendable section 1200 and the outer-catheter push section 1300 are not allowed to bend in a single direction, and their bending is resisted in different directions. Therefore, when under the action of equal pulling forces, the outer-catheter bendable section 1200 and the outer-catheter push section 1300 will bend in different planes. As a result, the outer-catheter bendable section 1200 and the outer-catheter push section 1300 are three-dimensionally bendable in a space. Referring to Fig. 3, the circumferential angle between the first and second axially bend-resistant portions 1210, 1310 may be in the range of 1° to 90°, such as 10°, 15°, 20°, 25°, 30°, 35°, 40°, 45°, 50°, 55°, 60°, 65°, 70°, 75°, 80°, 85°, 90°, or the like, and the present application is not limited to any particular such angle.

The outer-catheter bendable section 1200 and the outer-catheter push section 1300 may be separate structures, and a proximal end of the outer-catheter bendable section 1200 may be rotatably coupled to a distal end of the outer-catheter push section 1300. Based on a fixed-axis rotation between the outer-catheter bendable section 1200 and the outer-catheter push section 1300, the planar angle between the axial planes where the first and second axially bend-resistant portions 1210, 1310 are located can be adjusted, i.e., the aforementioned circumferential angle between the first and second axially bend-resistant portions 1210, 1310, and hence in a bending direction of the outer-catheter bendable section 1200 and the outer-catheter push section 1300 of the outer catheter 1000 in a three-dimensional space. With this arrangement, the outer catheter 1000 can be used in a wider range of applications for the treatment of various anatomically complex vascular lumens.

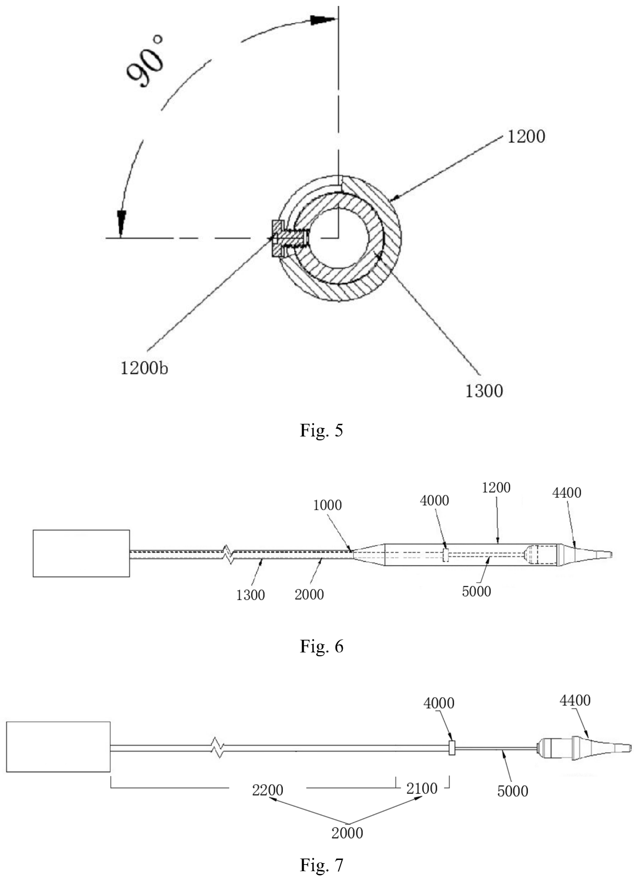

The rotatable coupling of the outer-catheter bendable section 1200 and the outer-catheter push section 1300 may be accomplished in various manners, for example, by a threaded connection, a snap-fit connection or the like. Referring to Figs. 4 and 5, in one embodiment, the proximal end of the outer-catheter bendable section 1200 and the distal end of the outer-catheter push section 1300 are provided with a pivot-adjusting slot 1200a and a mating pivot-locking element 1200b, respectively. The pivot-adjusting slot 1200a extends circumferentially, and the pivot-locking element 1200b is slidable along the pivot-adjusting slot 1200a to adjust an angle of fixed-axis rotation between the outer-catheter bendable section 1200 and the outer-catheter push section 1300. For example, the pivot-adjusting slot 1200a may be provided at the proximal end of the outer-catheter bendable section 1200, and the pivot-locking element 1200b may be provided at the distal end of the outer-catheter push section 1300. The distal end of the outer-catheter push section 1300 may be pivotably nested within the proximal end of the outer-catheter bendable section 1200 so that the pivot-locking element 1200b protrudes out through the pivot-adjusting slot 1200a. The outer-catheter bendable section 1200 and the outer-catheter push section 1300 may be pivotable relative to each other about the fixed axis, causing the pivot-locking element 1200b to slide along the pivot-adjusting slot 1200a. When a desired angle is reached, the outer-catheter bendable section 1200 and the outer-catheter push section 1300 may be locked against each other by the pivot-locking element 1200b.

The pivot-adjusting slot 1200a may circumferentially extend an angle of 1° to 90°. Without limitation, the pivot-locking element 1200b may be a snap-fit element, threaded element, or the like. Additionally, a sealing element 1200c, such as a sealing ring, may be provided between the proximal end of the outer-catheter bendable section 1200 and the distal end of the outer-catheter push section 1300 to prevent egress of blood during surgery through a gap between the outer-catheter bendable section 1200 and the outer-catheter push section 1300. For example, the pivot-locking element 1200b may include a fastening screw and a screw hole provided at the distal end of the outer-catheter push section 1300, and a sealing ring groove may also be provided at the distal end of the outer-catheter push section 1300. The pivot-adjusting slot 1200a may be provided at the proximal end of the outer-catheter bendable section 1200 as a guideway. In order to assemble them together, the distal end of the outer-catheter push section 1300 may be first inserted and nested into the proximal end of the outer-catheter bendable section 1200, and the fastening screw may be then inserted through the pivot-adjusting slot 1200a and pre-screwed into the screw hole slightly instead of tightly, in order to allow the fastening screw to move freely in the pivot-adjusting slot 1200a to enable smooth pivoting of the outer-catheter push and bendable sections 1300, 1200 relative to each other. The fastening screw may be always maintained in the same orientation as the second axially bend-resistant portion 1310 of the outer-catheter push section 1300. The sealing ring may serve to prevent egress of blood through the gap between the outer-catheter bendable section 1200 and the outer-catheter push section 1300 during surgery.

Prior to surgery, a surgeon or medical practitioner may pivot the outer-catheter bendable section 1200 as desired according to an image of a patient to adjust the angle of circumferential deflection between the first axially bend-resistant portion 1210 of the outer-catheter bendable section 1200 and the second axially bend-resistant portion 1310 of the outer-catheter push section 1300, and then tighten the fastening screw. In this way, the delivery system can be three-dimensionally bent at different angles under bending states so that a curved shape of the delivery system better conforms to an arch in the patient.

Further, in one embodiment, the outer-catheter bendable section 1200 and the outer-catheter push section 1300 may be an integrally formed structure, with the proximal end of the outer-catheter bendable section 1200 being fixed joined to the distal end of the outer-catheter push section 1300. In this case, the outer-catheter bendable section 1200 and the outer-catheter push section 1300 of the outer catheter 1000 are three-dimensionally bent relative to each other in a fixed direction. Accordingly, based on practical needs, catheters with different specifications are selected for surgical procedures.

The outer catheter 1000 may be used in a delivery system. For example, the outer catheter 1000 may be sleeved and assembled with an inner catheter 2000 to form a delivery component used in the delivery system. Referring to Figs. 6 and 7, the inner catheter 2000 may be movably mounted in an axial lumen of the outer catheter 1000 and bent by a pull wire 3000 or the like to in turn cause bending of the outer catheter 1000. With this arrangement, under the action of a bending force transferred from the inner catheter 2000 to the outer catheter 1000, due to the presence of a circumferential angular deflection between the first and second axially bend-resistant portions 1210, 1310 of the outer catheter 1000, as described above, the outer-catheter bendable section 1200 and the outer-catheter push section 1300 of the outer catheter 1000 will bend in different planes, enabling flexible three-dimensional bending of the outer catheter 1000 and smooth passage through lumens of different shapes in the body, such as the aortic arch. Moreover, as the angle of circumferential deflection and an active steering angle together determine a spatial orientation of a target object being delivered, such as a valve, a stent body or the like, based on active three-dimensional steerability of the outer catheter 1000, a valvular annulus in the same plane as the aortic arch can be maintained, facilitating coaxial adjustment and release of a valve stent, thereby significantly improving surgical outcomes.

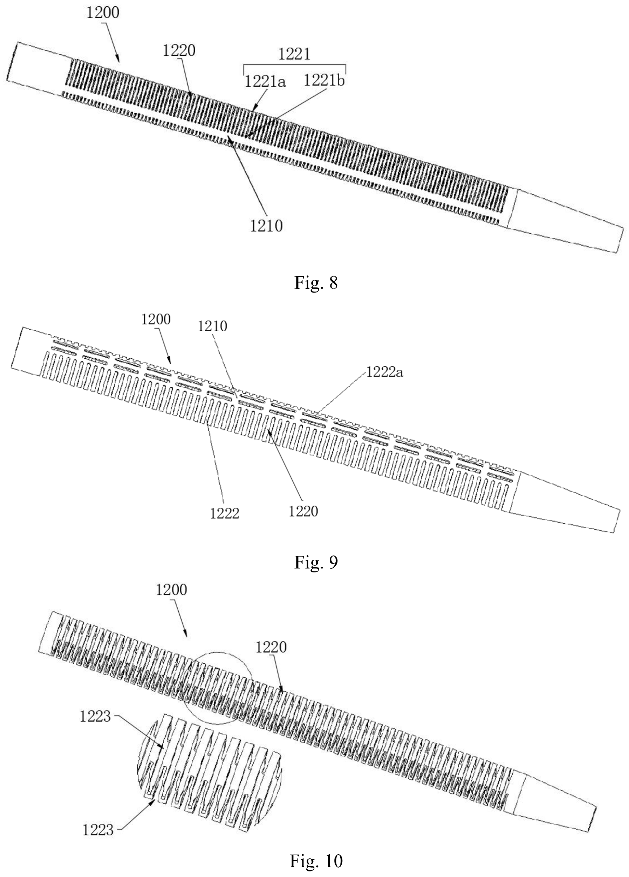

The outer-catheter bendable section 1200 may have one or more first axially bend-resistant portions 1210, as long as the bending direction of the outer-catheter bendable section 1200 can be restricted in a desired way. For example, as shown in Figs. 8 to 10, the outer-catheter bendable section 1200 may have two first axially bend-resistant portions 1210 that are symmetric about the central axis of the outer-catheter bendable section 1200. With this arrangement, the outer-catheter bendable section 1200 can bend only perpendicular to the axial planes where the two first axially bend-resistant portions 1210 are located. Similarly, the outer-catheter push section 1300 may have one or more second axially bend-resistant portions 1310, as long as the bending direction of the outer-catheter push section 1300 can be restricted in a desired way. For example, as shown in Figs. 8 to 10, the outer-catheter push section 1300 may have two second axially bend-resistant portions 1310 that are symmetric about the central axis of the outer-catheter push section 1300. With this arrangement, the outer-catheter push section 1300 can bend only perpendicular to the axial planes where the two second axially bend-resistant portions 1310 are located.

With continued reference to Figs. 8 to 10, in one embodiment, an outer-catheter bend-facilitating portion 1220 may be provided on the surface of the outer-catheter bendable section 1200 along the axis thereof. The outer-catheter bend-facilitating portion 1220 and the first axially bend-resistant portions 1210 may substantially span the entire surface of the outer-catheter bendable section 1200, and the present application is not limited to any particular proportions of the surface of the outer-catheter bendable section 1200 that they account for. The outer-catheter bend-facilitating portion 1220 may have no overlap with the axial planes of the first axially bend-resistant portions 1210 so that bending of the outer-catheter bendable section 1200 relies essentially on the outer-catheter bend-facilitating portion 1220 while being resisted by the first axially bend-resistant portions 1210. For example, the outer-catheter bend-facilitating portion 1220 may at least allow the outer-catheter bendable section 1200 to bend at a direction perpendicular to the axial planes where the first axially bend-resistant portions 1210 are located. Through different arrangements of the outer-catheter bend-facilitating portion 1220 and the first axially bend-resistant portions 1210 formed on the surface of the outer-catheter bendable section 1200, the outer-catheter bendable section 1200 can thus be configured with specific bendable directions. Without limitations, those skilled in the art may make designs according to their needs.

The outer-catheter bendable section 1200 may have one or more outer-catheter bend-facilitating portions 1220, as long as the outer-catheter bendable section 1200 can be made bendable in a desired direction. For example, as shown in Figs. 8 to 10, in one embodiment, the outer-catheter bendable section 1200 may have two outer-catheter bend-facilitating portions 1220 that are symmetric about the central axis of the outer-catheter bendable section 1200. With this arrangement, the outer-catheter bendable section 1200 is bendable due to the presence of the outer-catheter bend-facilitating portions 1220. The outer-catheter bend-facilitating portions 1220 may facilitate bending of the outer-catheter bendable section 1200 in various different ways. For example, the outer-catheter bend-facilitating portions 1220 of the outer-catheter bendable section 1200 may be made of an easy-to-bend material, or structured so as to be easily bendable, without limitations.

Referring to Fig. 8, in one embodiment, the outer-catheter bend-facilitating portions 1220 may include a plurality of first hollows 1221, each including a first arc-shaped slot 1221a and two first circular slots 1221b located at opposite ends of the first arc-shaped slot 1221a. The first arc-shaped slots 1221a may extend circumferentially and be perpendicular to the central axis of the outer-catheter bendable section 1200, and the first hollows 1221 may be arranged along the axis of the outer-catheter bendable section 1200. The first axially bend-resistant portions 1210 may be located between the two outer-catheter bend-facilitating portions 1220.

The first hollows 1221 may be formed by cutting a nickel-titanium alloy. The first hollows 1221 in the two outer-catheter bend-facilitating portions 1220 may use cutouts of the same shape, may be arranged symmetrical to each other and extend along an axis thereof. In this case, a region between the two outer-catheter bend-facilitating portions 1220 constitutes the first axially bend-resistant portion 1210, and the first axially bend-resistant portions 1210 are remaining regions of the surface of the outer-catheter bendable section 1200 that are not cut, which provide symmetrical backbones (the symmetrical outer-catheter bend-facilitating portions 1220). The two backbones impart both good compression resistance in the direction of their extension and excellent bendability in a direction perpendicular to them.

Two first circular slots 1221b are provided at opposite ends of the first arc-shaped slots 1221a that allow for greater stretching and compression of the outer-catheter bendable section 1200 during its bending and reduce the impact of the two backbones on the bending. As a result, the section can be more easily bent at a larger angle. Meanwhile, the opposite two backbones provide reinforcement and support, imparting increased stability to the outer-catheter bendable section 1200 by avoiding its twisting or wrinkling even when it is axially stretched or compressed.

With continued reference to Fig. 9, in one embodiment, each outer-catheter bend-facilitating portion 1220 may include a plurality of second arc-shaped slots 1222, which may extend circumferentially perpendicular to the central axis of the outer-catheter bendable section 1200 and be arranged along the axis of the outer-catheter bendable section 1200. In addition, the first bend-resistant portion 1210 may include at least two lines of axial straight holes 1222a extending along the axis. Bend-resistant backbones may be provided between adjacent lines of axial straight holes 1222a to resist bending of the outer-catheter bendable section 1200.

The second arc-shaped slots 1222 and the axial straight holes 1222a may be formed by cutting a nickel-titanium alloy. The second arc-shaped slots 1222 in the two outer-catheter bend-facilitating portions 1220 may use cutouts of the same shape, may be arranged symmetrical to each other and extend along an axis thereof. A region between the two outer-catheter bend-facilitating portions 1220 constitutes the first axially bend-resistant portion 1210, and the first axially bend-resistant portions 1210 are remaining regions of the surface of the outer-catheter bendable section 1200 that are not cut, which provide symmetrical backbones (the symmetrical outer-catheter bend-facilitating portions 1220). The at least two lines of axial straight holes 1222a extending along the axis may be provided in the remaining regions, and one line of axial straight holes1222a may consist of a plurality of axially spaced hole units.

With continued reference to Fig. 10, in one embodiment, the outer-catheter bend-facilitating portions 1220 may include a plurality of arc-shaped cutting grooves 1223, which extend circumferentially and is perpendicular to the central axis of the outer-catheter bendable section 1200 and are arranged along the axis of the outer-catheter bendable section 1200. The plurality of arc-shaped cutting grooves 1223 in the two outer-catheter bend-facilitating portions 1220 may be partially alternated along the axial direction. With particular reference to Fig. 10, the arc-shaped cutting grooves 1223 in one of the outer-catheter bend-facilitating portions 1220 may axially alternate with those in the other outer-catheter bend-facilitating portion 1220. That is, between the axially adjacent arc-shaped cutting grooves 1223 in the same outer-catheter bend-facilitating portion 1220, the arc-shaped cutting grooves 1223 in the other outer-catheter bend-facilitating portion 1220 may be provided.

The outer-catheter bendable section 1200 may have a greater diameter than the outer-catheter push section 1300. With this arrangement, when actively bending the outer-catheter bendable section 1200 by a pull wire 3000 or otherwise, the outer-catheter bendable section 1200 that is diametrically greater than the outer-catheter push section 1300 can produce a torque perpendicular to a cross-section of the outer-catheter bendable section 1200, thereby achieving a good steering control. That is, increasing the outer diameter of the outer-catheter push section 1300 with respect to the outer-catheter bendable section 1200 enable steering of the outer catheter 1000 at a larger angle. Referring to Fig. 6, in one embodiment, a proximal end of the outer-catheter bendable section 1200 may have a diameter-varying segment that is coupled to the distal end of the outer-catheter push section 1300. The diameter of the diameter-varying segment of the outer-catheter bendable section 1200 may gradually decrease from distal to proximal. Therefore, a transitional connection between the outer-catheter bendable section 1200 and the outer-catheter push section 1300 with different diameters is formed.

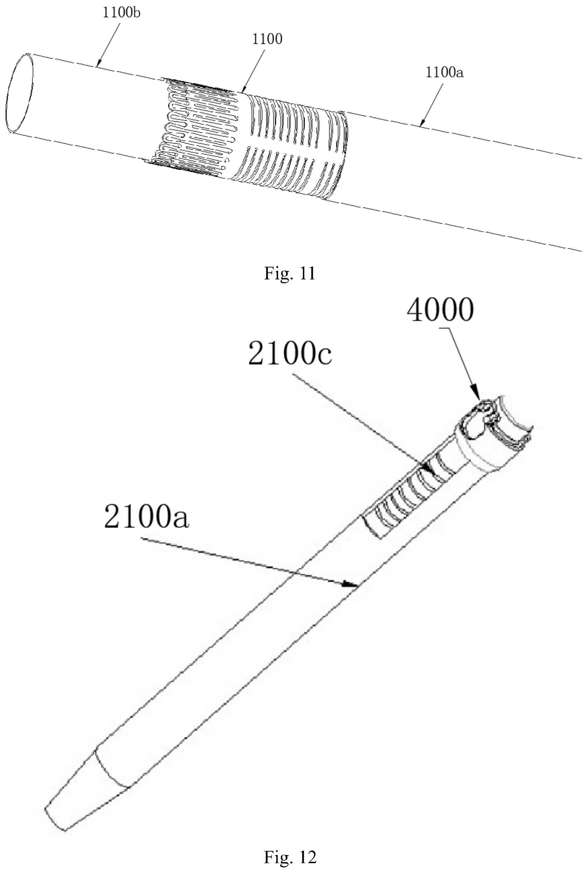

Referring to Figs. 11 and 12, the outer-catheter bendable section 1200 may include an outer-catheter outer layer 1100a and an outer-catheter inner layer 1100b. The main catheter body 1100 may be a reinforced metal tube obtained from a cutting process, which is made of, for example, stainless steel 316 or 304, a nickel-titanium alloy or the like. The outer-catheter outer layer 1100a may be provided with a polymetric coating layer, such as Pebax, polyamide (PA), thermoplastic silicone polyether polyurethane (TSPU), etc. The outer-catheter inner layer 1100b may also be provided with a polymetric coating layer, such as polytetrafluoroethylene (PTFE), high-density polyethylene (HDPE), etc. Those skilled in the art can select materials for those layers suited to practical needs, without limitations.

With continued reference to Figs. 6 and 7, also provided herein is a delivery component including the outer catheter 1000 as defined above, an inner catheter 2000, a pull wire 3000 and a stent body. The inner catheter 2000 comprises a guide lumen 2100d, and is movably sleeved within the axial lumen of the outer catheter 1000. The pull wire 3000 is coupled to at least one of the outer catheter 1000 and the inner catheter 2000. A valve is mounted on the stent body, and the stent body and the valve are configured to be loaded between the inner catheter 2000 and the outer catheter 1000. In one embodiment, the inner catheter 2000 may similarly include, along an axis thereof, an inner-catheter bendable section 2100 and an inner-catheter push section 2200. A proximal end of the inner-catheter bendable section 2100 is coupled to a distal end of the inner-catheter push section 2200. The inner-catheter bendable section 2100 may include an inner-catheter axially bend-resistant portion 2110.

The inner catheter 2000 may be movably sleeved in the axial lumen of the outer catheter 1000 and the inner catheter 2000 is axially movable relative to the outer catheter 1000 within the axial lumen thereof. The stent body and the valve thereon may be directly or indirectly mounted between the inner catheter 2000 and the outer catheter 1000. It should be noted that, as used hereinabove, the terms "axial direction" and "axially" refer to a direction along a central axis of the outer catheter 1000 or the inner catheter 2000, i.e., the left/right direction as shown in Figs. 6 and 7, and the terms "circumferential direction" and "circumferentially" refer to a direction about the central axis of the outer catheter 1000 or the inner catheter 2000.

The pull wire 3000 may be coupled to the outer catheter 1000 or the inner catheter 2000 at any desired location thereof. For example, in one embodiment, a distal end of the pull wire 3000 may be coupled to a distal end of the inner catheter 2000, for example, to a distal end of the inner-catheter bendable section 2100. Those skilled in the art may couple the pull wire 3000 to the inner-catheter bendable section 2100 at a location thereof, as required for desired steerability of the inner catheter 2000, or to the outer catheter 1000 if so desired, for example, to the outer-catheter bendable section 1200 of the outer catheter 1000, and the present application is not particularly limited in this regard.

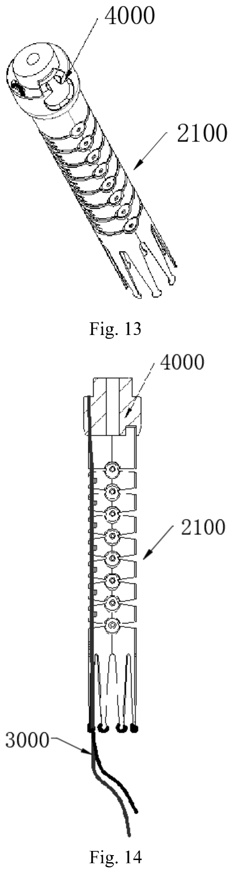

When a connection location of the pull wire 3000 and the inner catheter 2000 is determined, pulling the pull wire 3000 can apply a force to the inner catheter 2000 at said location to cause bending of the inner catheter 2000. The pull wire 3000 may be coupled to the inner catheter 2000 either directly or indirectly. For example, the coupling of the pull wire 3000 to the inner catheter 2000 may be accomplished by ligation, entwinement, welding or the like. Alternatively, referring to Figs. 6 and 13 to 15, a stent retainer 4000 may be provided on the inner catheter 2000, optionally at the distal end of the inner catheter 2000, for example, at the distal end of the inner-catheter bendable section 2100. Through the stent retainer 4000, the pull wire 3000 can be indirectly coupled to the inner catheter 2000. In the case, the location of the stent retainer 4000 on the inner catheter 2000 is a connection location of the pull wire 3000on the inner catheter 2000. The stent retainer 4000 may have a wire fastener 4200 configured for coupling of the pull wire 3000 thereto. The stent retainer 4000 may comprise an inner bore 4100 in communication with the guide lumen 2100d. A guide wire for guiding advancement of the delivery component in a direction may be passed through the inner bore 4100 and the guide lumen 2100d.

When the inner catheter 2000 is bent, for example, at the inner-catheter bendable section 2100, it will transmit the force to the outer catheter 1000, in turn causing bending of the outer catheter 1000. Due to presence of the circumferential angular deflection between the first and second axially bend-resistant portions 1210, 1310 of the outer catheter 1000, the outer-catheter bendable section 1200 and the outer-catheter push section 1300 of the outer catheter 1000 will bend in different planes, enabling flexible three-dimensional bending of the outer catheter 1000 and smooth passage through lumens of different shapes in the body, such as the aortic arch. Based on active three-dimensional steerability of the outer catheter 1000, a valvular annulus in the same plane as the aortic arch can be maintained, facilitating coaxial adjustment and release of a valve stent, thereby significantly improving surgical outcomes.

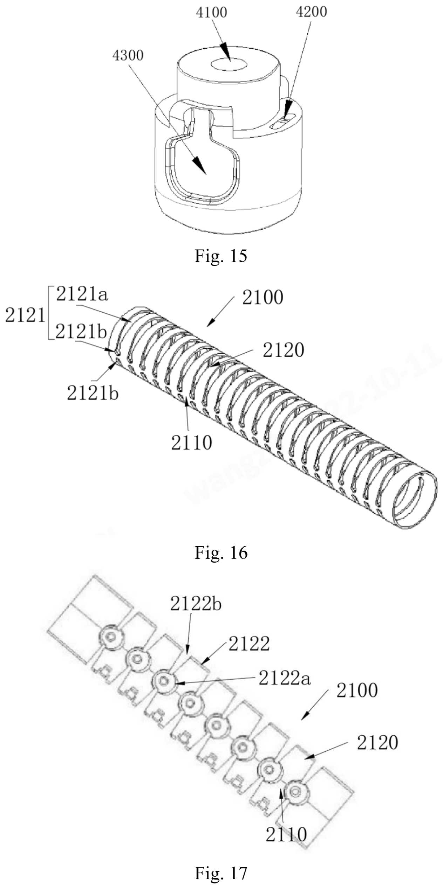

With continued reference to Fig. 6, the inner catheter 2000 may be coupled to an inner core tube 5000, which comprises a core-tube lumen, and proximal end of the inner catheter 2000 communicates with the distal end of the inner catheter 2000. A guide wire for guiding advancement of the delivery component in a direction may be passed through the guide lumen 2100d and the core-tube lumen. In this case, the stent body may be sleeved onto the inner core tube 5000 and thereby between the inner catheter 2000 and the outer catheter 1000. Referring to Fig. 15, the stent retainer 4000 may include a stent retention member 4300, which may comprise various different structures, such as a recess or through hole, as long as it enables fixation of the stent body. Additionally, in order to enable stable retention of the stent body, the recess or through bole may mate with connection structure of the stent body, such as a lug. Once the stent body and the valve thereon are retained, the stent retention member 4300 can restrict the stent body from moving, for example, circumferentially, axially or in other directions. The inner core tube 5000 may be provided at a distal end thereof with a distal guide member 4400, which may be tapered or otherwise structure to easily guide advancement of the entire delivery component.

The inner catheter 2000 may be bendable in any desired direction, or only in a specified direction predetermined as needed. For example, in one embodiment, the inner catheter 2000 may likewise include an inner-catheter axially bend-resistant portion 2110, which may extend along the axis of the inner catheter 2000 across its entire axial length or at least part thereof. The inner-catheter axially bend-resistant portion 2110 may be essentially in the form of an axially extending linear portion, and may have a width, which is determined as actually needed to enable the inner-catheter axially bend-resistant portion 2110 to resist bending of the inner catheter 2000 along an axial plane where the inner-catheter axially bend-resistant portion 2110 is located. The inner catheter 2000 may be straight when out of use and non-straight when in use. Accordingly, in the axially straight and non-straight configurations, the inner catheter 2000, and hence the axially extending inner-catheter axially bend-resistant portion 2110, may be linear trajectory or curved trajectory.

It should be noted that the aforementioned axial plane refers to a plane passing through a corresponding central axis. Specially, the axial plane where the inner-catheter axially bend-resistant portion 2110 is located passes through both a central axis and a surface of the inner catheter 2000, and by "resist bending of the inner catheter 2000 along an axial plane where the inner-catheter axially bend-resistant portion 2110 is located", it is intended to mean that bending of the inner catheter 2000 in the axial plane is made difficult, or even impossible at all.

The inner catheter 2000 may have one or more inner-catheter axially bend-resistant portions 2110, as long as the bending direction of the inner catheter 2000 can be restricted according to requirements. For example, as shown in Figs. 16 to 18, in one embodiment, the inner catheter 2000 may have two inner-catheter axially bend-resistant portions 2110, for example, both essentially in the inner-catheter bendable section 2100, which are symmetric about the central axis of the inner catheter 2000. With this arrangement, the inner catheter 2000 can bend at a direction only perpendicular to the axial planes of the two inner-catheter axially bend-resistant portions 2110.

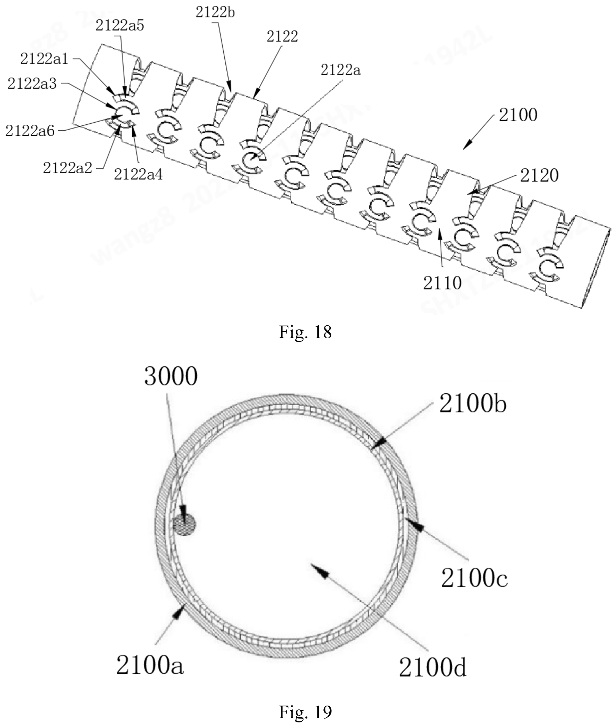

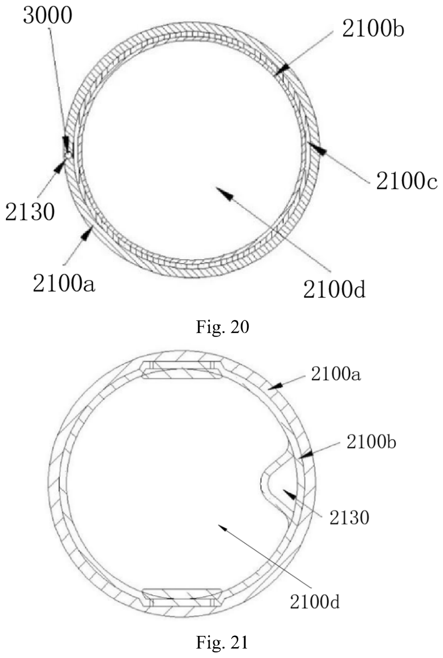

Pulling the inner catheter 2000 by the pull wire 3000 may be accomplished in various different ways. For example, in one embodiment, as shown in referring to Fig. 19, the pull wire 3000 may be threaded through the guide lumen 2100d of the inner catheter 2000. In this case, the pull wire 3000 will be freely arranged in the guide lumen 2100d, and when pulled, the tensioned pull wire 3000 will approach an inner wall surface of the guide lumen 2100d on the steered side. Alternatively, as shown in Figs. 20 and 21, at least one axial portion of the inner catheter 2000 may comprise a pulling channel 2130, and the pull wire 3000 may be threaded through the pulling channel 2130 of the inner catheter 2000. A perpendicular distance between central axes of the pulling channel 2130 and the inner catheter 2000 may be greater than a radius of the inner catheter 2000.

Referring to Figs. 20 and 21, the inner-catheter bendable section 2100 may include an inner-catheter outer layer 2100a, an inner-catheter inner layer 2100b and a reinforcing layer 2100c between the inner-catheter outer and inner layers 2100a, 2100b. The reinforcing layer 2100c may be a braided tube of stainless steel for medical use, or a reinforcing tube made of a metal material with tensile strength and easy bendability, such as a nickel-titanium alloy or stainless steel. The inner-catheter inner layer 2100b may be made of a low-friction material, such as PTFE or HDPE. The inner-catheter outer layer 2100a may be made of a polymetric material for medical use, such as Pebax or PA. The pulling channel 2130, and hence the pull wire 3000, may be arranged between the inner-catheter outer layer 2100a and the reinforcing layer 2100c. With this arrangement, steerability can be provided through applying a force to the distal end of the pull wire 3000. Alternatively, the pulling channel 2130 may be provided in an inner wall of the inner-catheter inner layer 2100b.

In one embodiment, the inner-catheter bendable section 2100 may include an axially extending inner-catheter bend-facilitating portion 2120, which does not have any overlap with the axial plane where the inner-catheter axially bend-resistant portion 2110 is located, allowing bending of the inner-catheter bendable section 2100 to rely essentially on the inner-catheter bend-facilitating portion 2120 while being resisted by the inner-catheter axially bend-resistant portion 2110. For example, the inner-catheter bend-facilitating portion 2120 may at least allow the inner-catheter bendable section 2100 to bend at a direction perpendicular to the axial plane where the inner-catheter axially bend-resistant portion 2110 is located. Through forming different distributions of the inner-catheter bend-facilitating portion 2120 and the inner-catheter axially bend-resistant portion 2110 on the surface of the inner-catheter bendable section 2100, the inner-catheter bendable section 2100 may comprise specific bending directions. Those skilled in the art may make designs according to their needs, without limitations.

The outer-catheter bendable section 1200 may have one or more outer-catheter bend-facilitating portions 1220, as long as the outer-catheter bendable section 1200 can be made bendable in a desired direction. For example, as shown in Figs. 16 to 18, in one embodiment, the inner-catheter bendable section 2100 may have two inner-catheter bend-facilitating portions 2120 symmetric about a central axis of the inner-catheter bendable section 2100. With this arrangement, the inner-catheter bendable section 2100 is bendable due to the presence of the inner-catheter bend-facilitating portions 2120. The inner-catheter bend-facilitating portions 2120 may facilitate bending of the inner-catheter bendable section 2100 in various different ways. For example, the inner-catheter bend-facilitating portions 2120 of the inner-catheter bendable section 2100 may be made of an easy-to-bend material, or structured so as to be easily bendable, without limitations.

Referring to Fig. 16, the inner-catheter bend-facilitating portions 2120 may include a plurality of second hollows 2121, each including a third arc-shaped slot 2121a and two second circular slots 2121b located at opposite ends of the third arc-shaped slot 2121a. The third arc-shaped slots 2121a may extend circumferentially and be perpendicular to the central axis of the inner-catheter bendable section 2100, and the plurality of second hollows 2121 may be arranged along the axis of the inner-catheter bendable section 2100. The regions between the two inner-catheter bend-facilitating portions 2120 constitute the inner-catheter axially bend-resistant portions 2110.

The second hollows 2121 may be formed by cutting a nickel-titanium alloy. The second hollows 2121 in the two inner-catheter bend-facilitating portions 2120 may use cutouts of the same shape, may be arranged symmetrical to each other and extend along an axis thereof. In this case, a region between the two inner-catheter bend-facilitating portions 2120 constitutes the inner-catheter axially bend-resistant portions 2110, and the inner-catheter axially bend-resistant portions 2110 are remaining regions of the surface of the inner-catheter bendable section 2100 that are not cut, which provide symmetrical backbones (the symmetrical inner-catheter axially bend-resistant portions 2110). The two backbones impart both good compression resistance in the direction of their extension and excellent bendability in a direction perpendicular to them.

Two opposite ends of the third arc-shaped slots 2121a comprise two second circular slots 2121b that allow for greater stretching and compression of the inner-catheter bendable section 2100 during its bending and reduce the impact of the two backbones on the bending. As a result, the section can be more easily bent at a larger angle. Meanwhile, the opposite two backbones provide reinforcement and support, imparting increased stability to the inner-catheter bendable section 2100 by avoiding its twisting or wrinkling even when it is axially stretched or compressed.

Referring to Figs. 17 and 18, the inner-catheter bendable section 2100 may include a plurality of joint units 2122 rotatably articulated along its axis and fixed-axis pivot parts 2122a between adjacent joint units 2122. The fixed-axis pivot parts 2122a may be located at the inner-catheter axially bend-resistant portions 2110, and with the aid of the axial arrangement of the fixed-axis pivot parts 2122a, resisting bending of the inner-catheter bendable section 2100 can be achieved. In addition, there may be rotation gaps 2122b between adjacent joint units 2122, the rotation gap 2122b is located at the inner-catheter bend-facilitating portions 2120. The presence of the rotation gaps 2122b enables bending of the inner-catheter bendable section 2100, optionally with overlap of adjacent joint units 2122 at the rotation gap 2122b.

With continued reference to Fig. 17, in one embodiment, the fixed-axis pivot part 2122a may include an axle and a hole in adjacent joint units 2122, and the joint units 2122 may be articulated so as to be rotatable about a fixed axis through the axle and hole. Alternatively, with continued reference to Fig. 18, the fixed-axis pivot part 2122a may include first and second rotatable engagement structures provided on respective corresponding adjacent joint units 2122. The first rotatable engagement structure may include a first arc-shaped slide slot 2122a1, a first arc-shaped fastening arm 2122a2 and a central fastening slot 2122a3, and the second rotatable engagement structure may include a second arc-shaped slide slot 2122a4, a second arc-shaped fastening arm 2122a5 and a central fastening head 2122a6. The first arc-shaped fastening arm 2122a2 may be slidably mounted along the second arc-shaped slide slot 2122a4, the second arc-shaped fastening arm 2122a5 may be slidably mounted along the first arc-shaped slide slot 2122a1, and the central fastening head 2122a6 is rotatably mounted in the central fastening slot 2122a3. The central fastening head 2122a6 and the central fastening slot 2122a3 will engage with each other when stressed, preventing the fixed-axis pivot part from opening.

Also provided herein is a delivery system comprising the outer catheter 1000 or the delivery component, as defined above. The structural details, functioning principles and technical effects of the outer catheter 1000 and the delivery component have been explained in detail above and will not be repeated here. Reference can be made to the foregoing description for any details of the outer catheter 1000 and the delivery component.

The various technical features of the foregoing embodiments may be combined in any way. Although not all such combinations have been described above for the sake of brevity, any of them is considered to fall within the scope of this specification as long as there is no contradiction between the technical features.

Presented above are some several embodiments of the present application. Although these embodiments are described with some particularity and in some detail, it should not be construed that they limit the scope of the present application in any sense. It is noted that various variations and modifications can be made by those of ordinary skill in the art without departing from the concept of the present application. Accordingly, it is intended that all such variations and modifications are embraced within the scope of this application as defined in the appended claims.

## Claims

1. An outer catheter for use in a delivery system, comprising:
a main catheter body comprising an axial lumen, wherein the main catheter body comprises an outer-catheter bendable section and an outer-catheter push section along an axial direction, wherein the outer-catheter bendable section comprises a first axially bend-resistant portion extending along the axial direction, wherein the first axially bend-resistant portion is configured to resist a bending of the outer-catheter bendable section along an axial plane where the first axially bend-resistant portion is located, wherein the outer-catheter push section comprises a second axially bend-resistant portion extending along the axial direction, wherein the second axially bend-resistant portion is configured to resist a bending of the outer-catheter push section along an axial plane where the second axially bend-resistant portion is located, and wherein an angle is formed between the axial plane where the first axially bend-resistant portion is located and the axial plane where the second axially bend-resistant portion is located.

2. The outer catheter according to claim 1, wherein the outer-catheter bendable section and the outer-catheter push section are an integrally formed structure, wherein a proximal end of the outer-catheter bendable section is fixedly joined to a distal end of the outer-catheter push section, or
wherein the outer-catheter bendable section and the outer-catheter push section are separate structures, wherein the proximal end of the outer-catheter bendable section is rotatably coupled to the distal end of the outer-catheter push section, and wherein based on a fixed-axis rotation between the outer-catheter bendable section and the outer-catheter push section, a planar angle between the axial planes where the first and second axially bend-resistant portions are located can be adjusted.

3. The outer catheter according to claim 2, wherein the proximal end of the outer-catheter bendable section and the distal end of the outer-catheter push section are provided with a pivot-adjusting slot and a mating pivot-locking element, respectively, wherein the pivot-adjusting slot extends circumferentially, wherein the pivot-locking element slides along the pivot-adjusting slot to adjust an angle of the fixed-axis rotation between the outer-catheter bendable section and the outer-catheter push section.

4. The outer catheter according to claim 1, wherein the outer-catheter bendable section has two first axially bend-resistant portions that are symmetric about a central axis of the outer-catheter bendable section, and/or
wherein the outer-catheter push section has two second axially bend-resistant portions that are symmetric about a central axis of the outer-catheter push section.

5. The outer catheter according to claim 1, wherein a surface of the outer-catheter bendable section comprises an outer-catheter bend-facilitating portion extending along the axial direction, wherein the outer-catheter bend-facilitating portion has no overlap with the axial plane where the first axially bend-resistant portion is located, and wherein the outer-catheter bend-facilitating portion is configured to at least allow the outer-catheter bendable section to bend along a direction perpendicular to the axial plane where the first axially bend-resistant portion is located.

6. The outer catheter according to claim 5, wherein the outer-catheter bendable section comprises two outer-catheter bend-facilitating portions that are in symmetric about a central axis of the outer-catheter bendable section.

7. The outer catheter according to claim 6, wherein the outer-catheter bend-facilitating portion is provided with a plurality of first hollows, wherein the first hollow comprises a first arc-shaped slot and two first circular slots located at opposite ends of the first arc-shaped slot, wherein the first arc-shaped slot extends circumferentially and is perpendicular to the central axis of the outer-catheter bendable section, wherein the plurality of the first hollows are arranged along the axial direction of the outer-catheter bendable section, and wherein a region between the two outer-catheter bend-facilitating portions constitutes the first axially bend-resistant portion.

8. The outer catheter according to claim 6, wherein the outer-catheter bend-facilitating portion is provided with a plurality of second arc-shaped slots that extends circumferentially and is perpendicular to the central axis of the outer-catheter bendable section, and wherein the plurality of second arc-shaped slots are arranged along the axial direction of the outer-catheter bendable section, and/or
wherein the first axially bend-resistant portion is provided with at least two lines of axial straight holes extending along the axial direction, wherein a bend-resistant backbone for resisting the bending of the outer-catheter bendable section is provided between the two lines of axial straight holes.

9. The outer catheter according to claim 8, wherein each line of axial straight hole comprises a plurality of hole units that are axially spaced apart.

10. The outer catheter according to claim 6, wherein the outer-catheter bend-facilitating portion is provided with a plurality of arc-shaped cutting grooves that extends circumferentially and is perpendicular to the central axis of the outer-catheter bendable section, wherein the plurality of arc-shaped cutting grooves are arranged along the axial direction of the outer-catheter bendable section, and wherein the plurality of arc-shaped cutting grooves in the two outer-catheter bend-facilitating portions are partially alternated along the axial direction.

11. The outer catheter according to claim 1, wherein a proximal end of the outer-catheter bendable section comprises a diameter-varying segment that is coupled to a distal end of the outer-catheter push section, wherein a diameter of the diameter-varying segment of the outer-catheter push section gradually decreases from a distal end to a proximal end, and/or
wherein the outer-catheter bendable section comprises an outer-catheter outer layer and an outer-catheter inner layer.

12. A delivery component, comprising:
the outer catheter of any one of claims 1 to 11;
an inner catheter comprising a guide lumen, wherein the inner catheter is movably mounted in an axial lumen of the outer catheter;
a pull wire coupled to at least one of the outer catheter and the inner catheter; and
a stent body mounted with a valve, wherein the stent body and the valve are configured to be mounted between the inner catheter and the outer catheter.

13. The delivery component according to claim 12, comprising:
a stent retainer provided at a distal end of the inner catheter; and/or
an inner core tube comprising a core-tube lumen, wherein a proximal end of the inner core tube communicates with the distal end of the inner catheter, wherein at least one of the guide lumen and the core-tube lumen is configured for passage of a guide wire therethrough.

14. The delivery component according to claim 13, wherein the stent retainer comprises an inner bore in communication with the guide lumen, and/or
wherein the stent retainer comprises a wire fastener, and/or
wherein the stent retainer comprises a stent retention member, and/or
wherein a distal guide member is provided at a distal end of the inner core tube.

15. The delivery component according to claim 12, wherein at least one axial section of the inner catheter comprises an inner-catheter axially bend-resistant portion extending along an axial direction, and wherein the inner-catheter axially bend-resistant portion is configured to resist a bending of the inner catheter along an axial plane where the inner-catheter axially bend-resistant portion is located.

16. The delivery component according to claim 15, wherein the inner catheter comprises two inner-catheter axially bend-resistant portions that are symmetric about a central axis of the inner catheter.

17. The delivery component according to claim 15, wherein the inner catheter comprises an inner-catheter bendable section and an inner-catheter push section along the axial direction, wherein a proximal end of the inner-catheter bendable section is coupled to a distal end of the inner-catheter push section, and wherein the inner-catheter axially bend-resistant portion is provided at least in the inner-catheter bendable section.

18. The delivery component according to claim 17, wherein a distal end of the pull wire is coupled to a distal end of the inner-catheter bendable section, or
wherein the pull wire is threaded through the guide lumen of the inner catheter, or
wherein at least portion of the inner catheter comprises a pulling channel, and wherein the pull wire is threaded through the pulling channel of the inner catheter.

19. The delivery component according to claim 17, wherein the inner-catheter bendable section comprises an inner-catheter outer layer, an inner-catheter inner layer and a reinforcing layer between the inner-catheter outer layer and the inner-catheter inner layer.

20. The delivery component according to claim 19, wherein a pulling channel is provided between the inner-catheter outer layer and the reinforcing layer, or wherein a pulling channel is provided in an inner wall of the inner-catheter inner layer.

21. The delivery component according to claim 17, wherein a surface of the inner-catheter bendable section comprises an inner-catheter bend-facilitating portion extending along the axial direction, wherein the inner-catheter bend-facilitating portion has no overlap with the axial plane where the inner-catheter axially bend-resistant portion is located, and wherein the inner-catheter bend-facilitating portion is configured to at least allow the inner-catheter bendable section to bend along a direction perpendicular to the axial plane where the inner-catheter axially bend-resistant portion is located.

22. The delivery component according to claim 21, wherein the inner-catheter bendable section is provided with two inner-catheter bend-facilitating portions that are symmetric about a central axis of the inner-catheter bendable section.

23. The delivery component according to claim 22, wherein the inner-catheter bend-facilitating portion comprises a plurality of second hollows comprising a third arc-shaped slot and two second circular slots located at opposite ends of the third arc-shaped slot, wherein the third arc-shaped slot extends circumferentially and is perpendicular to the central axis of the inner-catheter bendable section, and wherein the plurality of the second hollows are arranged along the axial direction of the inner-catheter bendable section, and wherein a region between the two inner-catheter bend-facilitating portions constitutes the inner-catheter axially bend-resistant portion.

24. The delivery component according to claim 22, wherein the inner-catheter bendable section comprises a plurality of joint units rotatably articulated along the axial direction, wherein a fixed-axis pivot part is provided between adjacent joint units, wherein the fixed-axis pivot part is located in the inner-catheter axially bend-resistant portion, wherein a rotation gap is provided between adjacent joint units, and wherein the rotation gap is provided in the inner-catheter bend-facilitating portions.

25. The delivery component according to claim 24, wherein the fixed-axis pivot part comprises an axle and a hole which are provided in adjacent joint units, or
wherein the fixed-axis pivot part comprises a first rotatable engagement structure and a second rotatable engagement structure which are provided on adjacent joint units, wherein the first rotatable engagement structure comprises a first arc-shaped slide slot, a first arc-shaped fastening arm and a central fastening slot, wherein the second rotatable engagement structure comprises a second arc-shaped slide slot, a second arc-shaped fastening arm and a central fastening head, wherein the first arc-shaped fastening arm is slidably mounted along the second arc-shaped slide slot, wherein the second arc-shaped fastening arm is slidably mounted along the first arc-shaped slide slot, and wherein the central fastening head is rotatably mounted in the central fastening slot.

26. A delivery system, comprising:
the outer catheter of any one of claims 1 to 11; or
the delivery component of any one of claims 12 to 25.
